# EUROPÄISCHE PATENTSCHRIFT

(11) **EP 0 337 417 B1**
(45) Veröffentlichungstag und Bekanntmachung des Hinweises auf die Patenterteilung: **09.02.1994**
(21) Anmeldenummer: 89106478.4
(22) Anmeldetag: 12.04.1989
(51) Int. Cl.: C07C 5/25, C07C 11/08, B01J 27/12

(54) **Verfahren zur Isomerisierung von n-Butenen**
Method for isomerising n-butenes
Procédé pour l'isomerisation des n-butènes

(30) Priorität: 15.04.1988 DE 3812515
(43) Veröffentlichungstag der Anmeldung: 18.10.1989
(73) Patentinhaber: Linde Aktiengesellschaft, 65189 Wiesbaden (DE)
(72) Erfinder: Zimmermann, Heinz, Dr. Ing., D-8000 München 71 (DE)
(74) Vertreter: Schaefer, Gerhard, Dr.

(56) Entgegenhaltungen:
- DE-A- 1 518 580
- DE-A- 1 518 596
- DE-B- 1 518 595
- US-A- 2 450 039

## Beschreibung

Die Erfindung betrifft ein Verfahren zur Isomerisierung von n-Butenen an Festbettkatalysatoren, die supersaure Fluorverbindungen der Elemente der III., IV., und/oder V. Hauptgruppe des Periodensystems enthalten.

Buten-1 ergibt bei der Alkylierung mit Isobutan zu hochoktanigen Benzinkomponenten schlechtere Produkte, d.h. Produkte mit einer niedrigeren Oktanzahl, als Buten-2. Aus diesem Grund sind Verfahren zur Isomerisierung von n-Butenen zu Buten-2 in der Raffinerietechnik von Bedeutung.

Butene werden im allgemeinen aus Crack-Verfahren (katalytisch oder mit Dampf) gewonnen, wobei ein Gemisch, welches im wesentlichen aus Buten-1, Buten-2 (cis und trans), Isobuten, n- und iso-Butan und Butadien besteht, gewonnen wird. Da Butadien und auch i-Buten als Rohstoffe gefragt sind, wird Butadien aus diesem Gemisch durch Extraktion und i-Buten z.B. in einer MTBE-Synthesestufe abgetrennt.

Ein bekanntes Verfahren zur Weiterverarbeitung eines derartigen C₄-Rohgasstromes wird beispielsweise in "hydrocarbon processing", September 1982, Nr. 9, Seite 176 beschrieben.

So ist die Isomerisierung von n-Butenen bislang nicht als eigenständige separate Reaktion durchgeführt worden, sondern lediglich als Neben- bzw. Folgereaktion der selektiven Polymerisation von Isobuten an sauren Multi-Festbettkatalysatoren.

Bei dem bekannten Verfahren erfolgt die Isomerisierung von Buten-1 zu Buten-2 mit Wasserstoff in einem der Polymerisation nachgeschalteten Festbettreaktor mit einem Hydroisomerisierungskatalysator.

Neben dem apparativen Aufwand durch die separate Reaktionsstufe ist auch der Zusatz von H₂ nachteilig, da Nebenprodukte wie z.B. Butan gebildet werden, was auch die Buten-2-Ausbeute negativ beeinflußt.

Aus der DE-A-15 18 580 ist die Isomerisierung von Butenen an einem Aluminiumoxid enthaltenden Katalysator mit 0,5 bis 1,5 Gew.% NH₄HF₂ bei Temperaturen von 250 bis 550° C und einer stündlichen Raumgeschwindigkeit (GHSV) von 100 bis 10 000 v/v bekannt.

Aus der DE-A-15 18 595 ist ein Verfahren zur Isomerisierung von Butenen an einem Aluminiumoxid enthaltenden Katalysator mit 0,5 bis 3,5 Gew.% SiF₆⁻⁻ bei Temperaturen von 250 bis 350° C und einer GHSV von 100 bis 10 000 v/v bekannt.

Aus der DE-A-15 18 580 ist ein Verfahren zur Isomerisierung von Butenen an einem Aluminiumoxid enthaltenden Katalysator mit 0,5 bis 2 Gew.% BF₄⁻ bei Temperaturen von 250 bis 550° C und einer GHSV von 100 bis 10 000 v/v bekannt.

In den drei genannten Dokumenten wird beschrieben, daß ein größerer Anteil an Fluorverbindungen im Katalysator als in den jeweils oben angeführten Bereichen zu hohen Anteilen an gecrackten und polymeren Produkten und daher zu wesentlichen Nachteilen für die erwünschte Butenisomerisierung führt bzw. daß bei höherem Anteil von Fluorverbindungen im Katalysator die Selektivität für die Umwandlung von Butenen in Folge übermäßiger Nebenreaktionen verhältnismäßig gering ist und damit eine geringe Isomerisierungsaktivität des Katalysators vorliegt.

Der vorliegenden Erfindung liegt daher die Aufgabe zugrunde, ein Verfahren der eingangs genannten Art so zu verbessern bzw. auszugestalten, daß auf einfache und kostengünstige Weise hohe Ausbeuten an Buten-2 bzw. Buten-1 erzielt werden können.

Diese Aufgabe wird dadurch gelöst, daß die Isomerisierung in der Gasphase an dem Katalysator, der aus einem Al₂O₃-Träger mit 5 bis 30 Gew.%, vorzugsweise 5 bis 15 Gew.%, supersauren Fluorverbindungen besteht, in Abwesenheit von Wasserstoff bei einer Temperatur von 50 bis 300°C durchgeführt wird.

Als supersaure Fluorverbindungen kommen alle Säuren in Frage, die Elemente der III., IV. und/oder V. Hauptgruppe des Periodensystems als Komponenten enthalten. Als Vertreter derartiger Supersäuren seien H₂SiF₆ und HPF₆ genannt. Mit gleichermaßen guten Ergebnissen können auch Salze dieser Supersäuren verwendet werden, wie beispielsweise (NH₄)₂SiF₆. Es ist klar, daß auch andere als die speziell genannten Säuren bzw. Salze zur Anwendung gelangen können.

Der erfindungsgemäß für die Isomerisierung eingesetzte Katalysator wird in an sich bekannter Weise durch Tränken des Trägers mit der Supersäure oder deren Salzlösungen und nachfolgender Calcinierung hergestellt, wobei die supersaure Komponente chemisch auf dem Träger gebunden wird.

Aufgrund der Trägerfixierung der Supersäure sind die Reaktionsmedien nicht korrosiv, was zusammen mit milden Reaktionsbedingungen zu günstigen Investitionskosten führt.

Unter Supersäuren werden dabei solche Säuren verstanden, die stärker sauer sind als eine 100%ige Schwefelsäure. Supersäuren besitzen in der Regel einen pH-Wert von etwa -0,5.

Die Auswahl von supersauren Fluorverbindungen der Elemente der III., IV. und/oder V. Hauptgruppe des Periodensystems hat sich als besonders günstig erwiesen, da mit einem derartig supersauren Katalysator hervorragende Ausbeuten bei hoher Selektivität und milden Reaktionsbedingungen erzielt werden können. Insbesondere kann die Isomerisierung bei Drücken von 1 bis 100 bar, mit Vorteil bei niedrigen Drücken von 1 bis 5 bar und einer stündlichen Gas-Raumgeschwindigkeit (gaseous hourly space velocity = GHSV) von 1000 bis 3000 l/h durchgeführt werden.

Die erfindungsgemäßen Reaktionsbedingungen ermöglichen in vorteilhafter Weise die Ausrichtung der Isomerisierung auf die Gewinnung von Buten-2 oder Buten-1.

Als besonders günstig im Hinblick auf die Buten-2-Bildung hat sich die Durchführung des erfindungsgemäßen Verfahrens bei einem Druck von 2 bar und einer Temperatur von 200°C und einer GHSV von 2000 l/h erwiesen, wobei sich diese günstigen Reaktionsbedingungen aus der hohen Aktivität des Katalysators ergeben.

Beispielsweise enthält das bei der Isomerisierung entstehende Produkt mindestens 80% Buten-2 bezogen auf umgesetztes Buten-1 womit dieses Produkt mit Vorteil direkt einer Alkylierung mit Isobutan zur Gewinnung hochoktaniger Benzinkomponenten zugeführt wird. Auf diese Weise lassen sich Alkylate, d.h. C₈-Benzine, mit einer Motoroktanzahl (MOZ) von 84 herstellen, wohingegen eine Alkylierung mit Buten-1 nur zu einer MOZ von 80 geführt hätte, was aufgrund des Verbots des Zusatzes von Bleitetraethyl einer minderwertiges Benzin ergibt.

Es hat sich gezeigt, daß bei Verwendung des erfindungsgemäßen Katalysators je nach den Reaktionsbedingungen das Isomerisierungsprodukt Buten-2 als ein Gemisch aus cis- und trans-Isomeren vorliegt.

Überdies ermöglicht das erfindungsgemäße Verfahren auch die Isomerisierung von cis- zu trans-Buten-2 ebenso wie die Isomerisierung von cis-Buten-2 zu Buten-1.

Als n-Buten-Einsatzgemische kommen C₄-Schnitte aus Crackgasen wie z.B. das Raffinat II infrage.

Reaktionen mit reaktiven Olefingemischen führen praktisch immer zur teilweisen Bildung fester Nebenprodukte, wie insbesondere Kohlenstoff oder Polymere, weshalb gemäß einer weiteren Ausgestaltung des erfindungsgemäßen Verfahrens der Katalysator von Zeit zu Zeit regeneriert wird, wobei dazu bevorzugt abgereicherte Luft durch das Katalysatorbett hindurchgeleitet wird.

Das erfindungsgemäße Verfahren ermöglicht es somit, auf einfache Weise - ohne gleichzeitige Polymerisation - Buten-1 zu Buten-2 zu isomerisieren um damit die Ausgangsbasis für eine kostengünstige Produktion von Alkylaten mit hoher MOZ zu schaffen.

Nachfolgend wird das erfindungsgemäße Verfahren anhand zweier Beispiele näher erläutert.

### Beispiel 1:

n-Buten-1 wird in einem Integralreaktor mit 20 ml Katalysatorvolumen (Katalysator 10% H₂SiF₆ auf Al₂O₃) mit einer GHSV von 2000 l/h bei 230°C bei Normaldruck isomerisiert. Das Reaktionsprodukt wies folgende Zusammensetzung auf:

| | |
|---|---|
| Buten-1 | 12,65 V% |
| trans Buten-2 | 51,93 V% |
| cis Buten-2 | 34,93 V% |
| Isobuten | 0,21 V% |

Hieraus ergibt sich ein Umsatz von 87,35%, bezogen auf Buten-1.

### Beispiel 2:

cis-Buten 2 wird bei 240°C und einer GHSV von 3000 l/h in einemIntegralreaktor mit 20 ml Katalysator (10% H₂SiF₆ auf Al₂O₃) bei Normaldruck isomerisiert. Hierbei wird folgendes Produkt erhalten:

| | |
|---|---|
| Buten-1 | 11,82 V% |
| trans Buten-2 | 35,97 V% |
| cis Buten-2 | 52,01 V% |
| Isobuten | 0,07 V% |

Hieraus ergibt sich ein Umsatz von 47,99%, bezogen auf cis Buten-2.

## Patentansprüche

1. Verfahren zur Isomerisierung von n-Butenen an Festbettkatalysatoren, die supersaure Fluorverbindungen der Elemente der III., IV., und/oder V. Hauptgruppe des Periodensystems enthalten, **dadurch gekennzeichnet**, daß die Isomerisierung in der Gasphase an dem Katalysator, der aus einem Al₂O₃-Träger mit 5 bis 30 Gew.%, vorzugsweise 5 bis 15 Gew.%, supersauren Fluorverbindungen besteht, in Abwesenheit von Wasserstoff bei einer Temperatur von 50 bis 300° C durchgeführt wird.

2. Verfahren nach Anspruch 1, dadurch gekennzeichnet, daß als supersaure Fluorverbindungen die Supersäuren HPF₆ oder H₂SiF₆ oder das Salz der letzgenannten Supersäure (NH₄)₂SiF₆ verwendet werden.

3. Verfahren nach einem der Ansprüche 1 oder 2, dadurch gekennzeichnet, daß die Isomerisierung bei einem Druck von 1 bis 100 bar, vorzugsweise 1 bis 5 bar und einer stündlichen Gas-Raumgeschwindigkeit (GHSV) von 1000 bis 3000 l/h durchgeführt wird.

4. Verfahren nach einem der Ansprüche 1 bis 3, dadurch gekennzeichnet, daß die Isomerisierung bei einem Druck von 2 bar und einer Temperatur von 200° C und einer GHSV von 2000 l/h durchgeführt wird.

5. Verfahren nach einem der Ansprüche 1 bis 4, dadurch gekennzeichnet, daß der Katalysator von Zeit zu Zeit regeneriert wird.

6. Verfahren nach Anspruch 5, dadurch gekennzeichnet, daß der Katalysator mit abgereicherter Luft regeneriert wird.

7. Verfahren nach einem der Ansprüche 1 bis 6, dadurch gekennzeichnet, daß die Isomerisierung auf die Gewinnung von Buten-2 ausgerichtet wird.

8. Verfahren nach einem der Ansprüche 1 bis 7, dadurch gekennzeichnet, daß das bei der Isomerisierung entstehende Produkt direkt einer Alkylierung mit Isobutan zur Gewinnung hochoktaniger Benzinkomponenten zugeführt wird.

9. Verfahren nach einem der Ansprüche 1 bis 8, dadurch gekennzeichnet, daß die Isomerisierung auf die Gewinnung von Buten-1 ausgerichtet wird.

## Claims

1. A process for the isomerization of n-butenes in the presence of fixed-bed catalysts which contain super acid fluorine compounds of the elements of the IIIrd, IVth and/or Vth main groups of the periodic system, characterised in that the isomerization is carried out in the gas phase in the presence of the catalyst which consists of a Al₂O₃ carrier containing 5 to 30 % by weight, preferably 5 to 15 % by weight, super acid fluorine compounds, in the absence of hydrogen at a temperature of 50 to 300°C.

2. A process as claimed in Claim 1, characterised in that the super acids HPF₆ or H₂SiF₆ or the salt of the last mentioned super acid (NH₄)₂SiF₆ are used as super acid fluorine compounds.

3. A process as claimed in one of Claims 1 or 2, characterised in that the isomerization is carried out at a pressure of 1 to 100 bar, preferably 1 to 5 bar, and at a gaseous hourly space velocity (GHSV) of 1000 to 3000 l/h.

4. A process as claimed in one of Claims 1 to 3, characterised in that the isomerization is carried out at a pressure of 2 bar and at a temperature of 200°C and at a GHSV of 2000 l/h.

5. A process as claimed in one of Claims 1 to 4, characterised in that the catalyst is periodically regenerated.

6. A process as claimed in Claim 5, characterised in that the catalyst is regenerated with depleted air.

7. A process as claimed in one of Claims 1 to 6, characterised in that the isomerization is adapted to the acquisition of 2-butene.

8. A process as claimed in one of Claims 1 to 7, characterised in that the product obtained by the isomerization is conveyed directly to an alkylation stage using isobutane for the acquisition of high-octane gasoline components.

9. A process as claimed in one of Claims 1 to 8, characterised in that the isomerization is adapted to the acquisition of 1-butene.

## Revendications

1. Un procédé d'isomérisation, sur des catalyseurs en lit fixe, de n-butène contenant des composés fluorés superacides des éléments des groupes III, IV et/ou V de la Classification Périodique, caractérisé en ce que l'isomérisation est mise en oeuvre en phase gazeuse sur un catalyseur, qui consiste en un support d'Al₂O₃ avec 5 à 30% en poids, et de préférence de 5 à 15% en poids, de composés fluorés superacides, en l'absence d'hydrogène et à une température de 50°C à 300°C.

2. Le procédé selon la revendication 1, caractérisé en ce que l'on utilise en tant que composé fluoré superacide des superacides HPF₆ ou H₂SiF₆ ou le sel de ce dernier acide (NH₄)₂SiF₆.

3. Le procédé selon la revendication 1 à 3, caractérisé en ce que l'isomérisation est mise en oeuvre sous une pression de 1 à 100 bars, de préférence 1 à 5 bar, et avec une vitesse spatiale horaire volumique (VSHV) de 1000 à 3000 litres/heure.

4. Le procédé selon l'une quelconque des revendications 1 à 3, caractérisé en ce que l'isomérisation est mise en oeuvre sous une pression de 2 bar à une température de 200°C et une VSHV de 2000 litres/heure.

5. Le procédé selon l'une quelconque des revendications 1 à 4, caractérisé en ce que le catalyseur est régénéré périodiquement.

6. Le procédé selon la revendication 5, caractérisé en ce que le catalyseur est régénéré avec de l'air enrichi.

7. Le procédé selon l'une quelconque des revendications 1 à 6, caractérisé en ce que l'isomérisation est dirigée vers la production de butène-2.

8. Le procédé selon l'une quelconque des revendications 1 à 7, caractérisé en ce que le produit obtenu par isomérisation est envoyé directement vers une étape d'alkylation avec de l'isobutane pour la production d'essences à indice d'octane élevé.

9. Le procédé selon l'une quelconque des revendications 1 à 8, caractérisé en ce que l'isomérisation est dirigée vers la production de butène-1.
